# EUROPÄISCHE PATENTANMELDUNG

(11) **EP 2 264 411 A1**
(43) Veröffentlichungstag der Anmeldung: **22.12.2010**
(21) Anmeldenummer: 09007810.6
(22) Anmeldetag: 15.06.2009
(51) Int. Cl.: G01G 19/414, A61B 5/053

(54) **Personenwaage mit Körpfett-Messvorrichtung**

(71) Anmelder: Beurer GmbH, 89077 Ulm (DE)
(72) Erfinder: Bühler, Marco, 89077 Ulm (DE); Meternek, Werner, 89233 Neu-Ulm (DE); Merk, Ernst, 89264 Weißenhorn (DE); Hörger, Marion, 89185 Hüttisheim (DE); Hafner, Heiko, 89233 Neu-Ulm (DE)
(74) Vertreter: Fleck, Hermann-Josef

(57) **Zusammenfassung**

Die Erfindung bezieht sich auf eine Personenwaage mit einer Messvorrichtung für einen Körperfettanteil, die eine Impedanzmesseinrichtung aufweist, wobei der Körper mit einem Messstrom, insbesondere einem einen Wechselanteil enthaltenden Strom beaufschlagt wird und eine Fußmesseinheit (20) dadurch gebildet ist, dass auf einer Messplattform mindestens zwei Fußelektroden (21, 21') zum Einspeisen des Stroms in den Körper angeordnet sind. Eine erhöhte Bedienerfreundlichkeit wird dadurch erreicht, dass die Messvorrichtung des Weiteren eine Handmesseinheit (10) mit mindestens zwei Handelektroden (11, 11') aufweist, wobei eine Auswerteeinrichtung (30) zur Verarbeitung der von den Fußelektroden (21, 21') und von den Handelektroden (11, 11') erhaltenen Signale ausgebildet ist und die Signale von den Fußelektroden (21, 21') und/oder den Handelektroden (11, 11') drahtlos an die Auswerteeinrichtung (30) übertragen werden (Fig. 1).

## Beschreibung

Die Erfindung bezieht sich auf eine Personenwaage mit einer Messvorrichtung für einen Körperfettanteil, die eine Impedanzmesseinrichtung aufweist, wobei der Körper mit einem Messstrom, insbesondere einem einen Wechselanteil enthaltenden Strom beaufschlagt wird und eine Fußmesseinheit dadurch gebildet ist, dass auf einer Messplattform mindestens zwei Fußelektroden zum Einspeisen des Stroms in den Körper angeordnet sind.

Bekannte Körperfettwagen dieser Art weisen, wie z. B. die US 6,308,096 B1 und die US 6,321,112 B1 zeigen, meist eine Messplattform für die Gewichtsmessung einer Person auf, auf deren Oberfläche zwei getrennte Elektroden aufgebracht sind, auf welchen die Füße der auf der Messplattform stehenden Person positioniert sind. Der Körperfettanteil wird mittels einer Impedanzmessung bei einer bestimmten Wechselspannung bzw. des daraus resultierenden, durch den Körper der Person fließenden Stroms bei einer bestimmten Frequenz ermittelt. Die Körperfettmessung wird dabei durch Handmesselektroden unterstützt, wobei ein Messstrom über Verbindungsleitungen auch zwischen den Hand- und Fußelektroden bewirkt sind. Körperfettmessungen mit Fuß- und Handelektroden sind auch in der EP 1 568 318 A1 genannt.

Der Körperfettmessung liegt die Erkenntnis zugrunde, dass der Körperwiderstand eines Lebewesens als ein dielektrischer Widerstand betrachtet werden kann, der auch vom Fettgehalt, Wasseranteil bzw. Muskelanteil und dgl. des Körpers abhängig ist, so dass durch eine Bioimpedanzanalyse (BIA-Messung) unter Beaufschlagung des Körpers über geeignete Elektroden mit Wechselstrom auf den Körperfettanteil, den Wasseranteil usw. rückgeschlossen werden kann.

Der Erfindung liegt die Aufgabe zugrunde, eine Körperfettwaage bereit zu stellen, die bedienerfreundlich aufgebaut ist.

Diese Aufgabe wird erfindungsgemäß dadurch gelöst, dass die Messvorrichtung des Weiteren eine Handmesseinheit mit mindestens zwei Handelektroden aufweist, wobei eine Auswerteeinrichtung zur Verarbeitung der von den Fußelektroden und von den Handelektroden erhaltenen Signale ausgebildet ist und die Signale von den Fußelektroden und/oder den Handelektroden drahtlos an die Auswerteeinrichtung übertragen werden.

Mittels der Anordnung der Handelektroden an der Handmesseinheit werden zusammen mit der Fußmesseinheit und der drahtlosen Übertragungsverbindung zwei in der Handhabung unabhängige Einheiten erhalten, die eine ungehinderte Benutzung der Waage durch den Bediener und auch Vorteile bei der Aufbewahrung ergeben. Der Messstrom kann dabei im einfachen Fall ein reiner Gleichstrom sein; besser für die Auswertung ist aber ein Wechselanteil, der ein reiner Wechselstrom oder ein einem Gleichstrom überlagerter Wechselstrom sein kann.

Für die Funktion sind dabei die Maßnahmen von Vorteil, dass zur Messung ein Wechselstrom mit mindestens zwei Frequenzen zwischen 1 kHz und 1000 kHz, insbesondere zwischen 3 kHz und 70 kHz verwendet wird. Dabei ist z. B. ein Frequenzbereich zwischen 5 kHz und 50 kHz vorteilhaft nutzbar.

Erweiterte Verwendungsmöglichkeiten ergeben sich dadurch, dass sie auch für die Messungen der Knochenmasse, der Muskelmasse und/oder des Wasseranteils ausgebildet ist.

Zu einer zuverlässigen Messung tragen die Maßnahmen bei, dass die Impedanzmesseinrichtung in Mehrleiter-, insbesondere Vierleitertechnik ausgebildet ist. Möglich sind aber auch eine Zwei- oder eine andere Mehrleitermesstechnik.

Für die Auswertung der Messung und Bestimmung der durch die Handmesseinheit und Fußmesseinheit jeweils gemessenen Körperfettwerte sind die Maßnahmen von Vorteil, dass die Messungen mit den Handelektroden und mit den Fußelektroden zueinander zeitversetzt erfolgen.

Verschiedene Ausgestaltungsvarianten bestehen darin, dass die drahtlose Verbindung als Funkverbindung, Ultraschallverbindung, Infrarotverbindung oder mit Transpondertechnik oder als Kombination mindestens zweier dieser Verbindungen ausgebildet ist.

Nähere Aussagen zum Körperfettgehalt werden dadurch erhalten, dass die Messvorrichtung zur Messung von Teil-Körperfettwerten einerseits mit der Fußmesseinheit und andererseits mit der Handmesseinheit ausgebildet ist.

Eine weitere vorteilhafte Ausgestaltung besteht darin, dass mit der Auswerteeinrichtung aus den Teil-Körperfettwerten ein Gesamt-Körperfettwert berechenbar ist, und dass der Gesamt-Körperfettwert und die Teil-Körperfettwerte mittels einer Anzeigevorrichtung anzeigbar sind.

Zu einer vorteilhaften Funktionsweise tragen die Maßnahmen bei, dass die Auswerteeinrichtung eine Auswerteeinheit aufweist, die zum Ermitteln und Bewerten eines ohmschen Widerstandsanteils und eines Blindwiderstandsanteils bei mindestens zwei verschiedenen Frequenzen sowohl bei der Messung mit der Handmesseinheit als auch mit der Fußmesseinheit ausgebildet ist.

Dabei besteht eine vorteilhafte Ausgestaltung zum Ermitteln der Messwerte darin, dass mittels der Auswerteeinheit die Teil-Körperfettwerte und/oder der Gesamtkörperfettwert in Abhängigkeit der ohmschen Anteile und der Blindwiderstandsanteile ermittelt werden.

Zum Bedienungskomfort trägt die Maßnahme bei, dass eine Anzeigeeinheit der Anzeigevorrichtung in der Handmesseinheit angeordnet ist.

Vorzugsweise werden bei der Durchführung der Messung mehrere verschiedene Frequenzen gleichzeitig oder nacheinander gewählt.

Die Erfindung wird nachfolgend anhand von Ausführungsbeispielen unter Bezugnahme auf die Zeichnungen näher erläutert. Es zeigen:
- Fig. 1: eine schematische Darstellung mit einem Ersatzschaltbild von Körper- impedanzen aus kapazitiven und ohmschen Widerstandsanteilen,
- Fig. 2: eine schematische Darstellung der Personenwaage mit Körperfett- Messvorrichtung aus Handteil und Fußteil und

- Fig. 3A und 3B: eine Anzeigevorrichtung mit zwei verschiedenen Anzeigeinhalten.

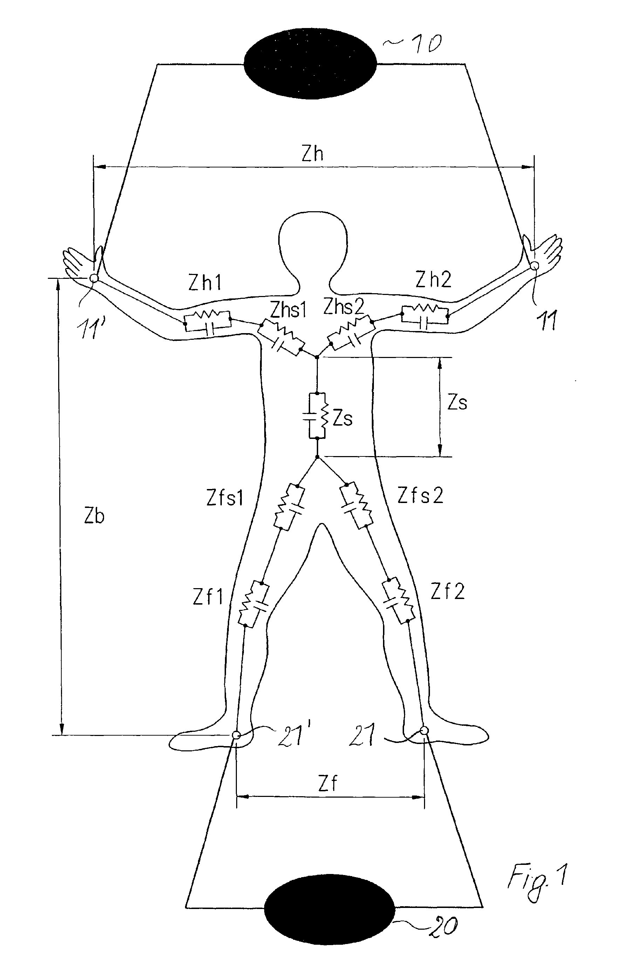

Fig. 1 zeigt schematisch einen menschlichen Körper mit Darstellung des Körperwiderstandes aus mehreren Teilwiderständen, die einen ohmschen und dazu parallel liegenden kapazitiven Widerstandsanteil aufweisen. Den Händen des menschlichen Körpers sind eine rechte und eine linke Handelektrode 11, 11' zugeordnet, die an eine Handmesseinheit 10 angeschlossen sind, während den Füßen eine rechte und linke Fußelektrode 21, 21' zugeordnet sind, die an eine Fußmesseinheit 20 angeschlossen sind. Über die Handmesseinheit 10 wird an die Handelektroden 11, 11' eine Spannung zum Erzeugen eines Messstroms zwischen den Handelektroden angelegt, während an die Fußelektroden 21, 21' eine Spannung zum Erzeugen eines Messstroms zwischen den Fußelektroden 21, 21' angelegt wird. Die Spannung ist eine Wechselspannung oder eine einer Gleichspannung überlagerte Wechselspannung, wobei die Wechselspannung bzw. der Wechselanteil eine Frequenz im Bereich zwischen 1 kHz und 1000 kHz, insbesondere zwischen 3 kHz und 70 kHz, vorzugsweise zwischen 5 kHz und 50 kHz besitzt. Zwischen den Fußelektroden 21, 21' besteht ein frequenzabhängiger Widerstand bzw. eine Impedanz Zf, die sich aus mehreren Teilimpedanzen Zf1, Zf2, Zfs1, Zfs2 zusammensetzt, während zwischen den Handelektroden 11, 11' eine Impedanz Zh vorliegt, die sich aus Teilimpedanzen Zh1, Zh2 und Zhs1, Zhs2 zusammensetzt. Dabei ist im Übergangsbereich zwischen den Teilimpedanzen zwischen den Handelektroden 11, 11' bzw. des Oberkörpers und den Teilimpedanzen zwischen den Fußelektroden 21, 21' bzw. des Unterkörpers noch eine Körperimpedanz Zs im Rumpfbereich vorhanden, der die Impedanzmessungen über die Fußelektroden und die Handelektroden mit beeinflusst und insbesondere auch bei der Gesamtkörperimpedanz Zb zur Auswirkung kommt.

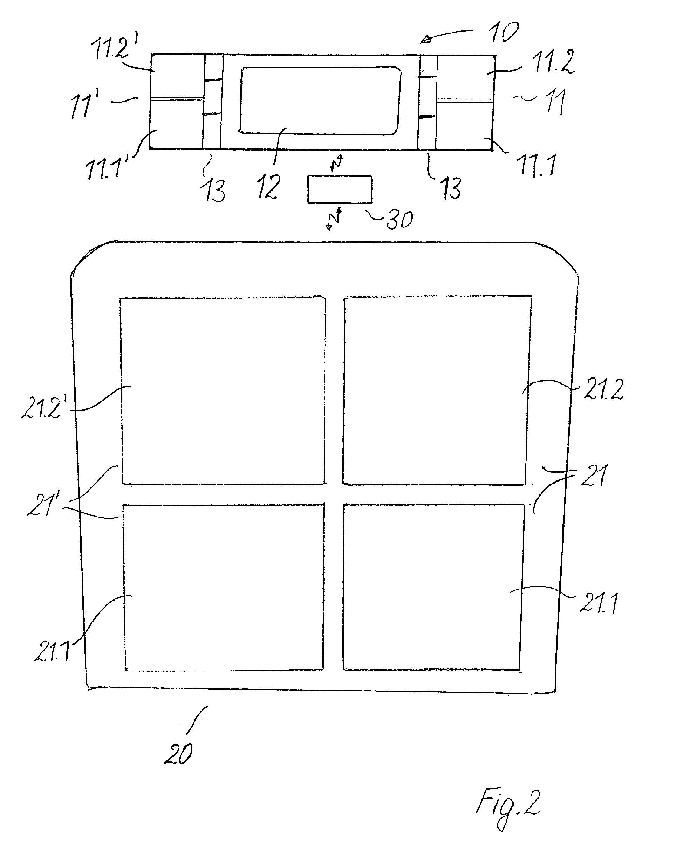

Fig. 2 zeigt die Handmesseinheit 10 mit den Handelektroden 11, 11' und die Fußmesseinheit 20 mit den Fußelektroden 21, 21', die im Betrieb in eine drahtlose Datenübertragungsverbindung miteinander gebracht sind und dazu jeweilige Schnittstellen zur drahtlosen Signalübertragung aufweisen. Allerdings fließt über die drahtlose Verbindung kein Messstrom, so dass sich also keine Messströme zwischen den Fuß-und Handelektroden ergeben. Die Elektroden der Handmesseinheit 11, 11' sind jeweils in zwei Teilelektroden 11.1, 11.2 bzw. 11.1', 11.2' unterteilt, um eine an sich bekannte Vierleitermessvorrichtung zu realisieren.

Weiterhin weist die Handmesseinheit eine Anzeigeeinheit 12, z. B. eine Flüssigkristallanzeigeeinheit oder dgl. auf, mit der alphanumerische und/oder graphische Informationsanzeigen, insbesondere der Messergebnisse erfolgen. Ferner sind Bedienelemente 13, beispielsweise Eingabetasten, an der Handmesseinheit 10 angeordnet.

Die Fußelektroden 21, 21' sind vorliegend ebenfalls jeweils als zwei Teilelektroden 21.1, 21.2 bzw. 21.1', 21.2' ausgebildet, um auch im Bereich der Fußmesseinheit 20 eine Vierleitermesstechnik zu realisieren. Die Vierleitermesstechnik ist vom Aufbau her zwar aufwändiger als die Zweileitermesstechnik, ermöglicht aber genauere Messungen.

Zur Verarbeitung der Messergebnisse der Fußmesseinheit 20 und der Handmesseinheit 10 weist die Messvorrichtung mit der Impedanzmesseinrichtung eine Auswerteeinrichtung 30 auf, der die Messsignale der Handmesseinheit 10 und/oder der Fußmesseinheit 20 drahtlos, z. B. über eine Funkverbindung, Ultraschallverbindung, Infrarotverbindung oder mittels Transpondertechnik zugeführt werden. Die Auswerteeinrichtung 30 ist vorteilhaft in der Handmesseinheit 10 oder alternativ in der Fußmesseinheit 20 angeordnet, wodurch lediglich eine Schnittstelle für die drahtlose Datenübertragung an der Fußmesseinheit 20 oder der Handmesseinheit 10 vorhanden sein müssen, die vorteilhaft für eine bidirektionale Datenübertragung ausgebildet sind. Die Messvorrichtung, und zwar beispielsweise die Auswerteeinrichtung 30, ist vorteilhaft mit flüchtigen und/oder permanenten Speichervorrichtungen versehen, um die Messdaten permanent und/oder vorübergehend zu speichern.

Zum Erfassen der Körperfettwerte werden die Messungen mit der Handmesseinheit 10 und der Fußmesseinheit 20 vorzugsweise zeitlich versetzt durchgeführt, so dass die Messsignale zeitlich voneinander getrennt sind und eindeutig den Handelektroden 11, 11' und Fußelektroden 21, 21' zuordenbar sind. Ferner werden Messungen bei mindestens zwei unterschiedlichen Frequenzen durchgeführt und die Messsignale hinsichtlich ihres ohmschen Widerstandsanteils und ihres Blindwiderstandsanteils, insbesondere kapazitiven Widerstandsanteils, ausgewertet, da das Verhältnis von ohmschem Widerstandsanteil und Blindwiderstandsanteil bei verschiedenen Frequenzen unterschiedlich ist. Hierdurch können Rückschlüsse gezogen werden, in welchem Ausmaß die verschiedenen Körperbereiche die Messung des Fettanteils beeinflussen bzw. mit beeinflussen. Hierbei können die eingespeisten Wechselspannungssignale bzw. Wechselstromsignale frequenzsynchron und/oder frequenzasynchron zueinander stehen und/oder Phasenverschiebungen und/oder feste oder variable Amplituden der Spannungen und damit zusammenhängenden Messsignale berücksichtigt werden. Aus den verschiedenen bekannten eingespeisten Wechselspannungen und den gemessenen Wechselspannungen mit ihrer jeweiligen Frequenzbeziehung, Phasenbeziehung und/oder Amplitudenbeziehung können rechnerisch die Körperfettanteile ermittelt werden, woraus für den oberen Körperbereich einerseits und den unteren Körperbereich andererseits ermittelt und daraus zudem der Gesamt-Körperfettwert bestimmt werden können. Auch können Berechnungen für Körperfettwerte im linken und/oder rechten Körperbereich durchgeführt werden. Neben dem Körperfettanteil können auch der Körperwasseranteil und/oder der Körpermuskelmasseanteil und/oder der Knochenmasseanteil ermittelt werden.

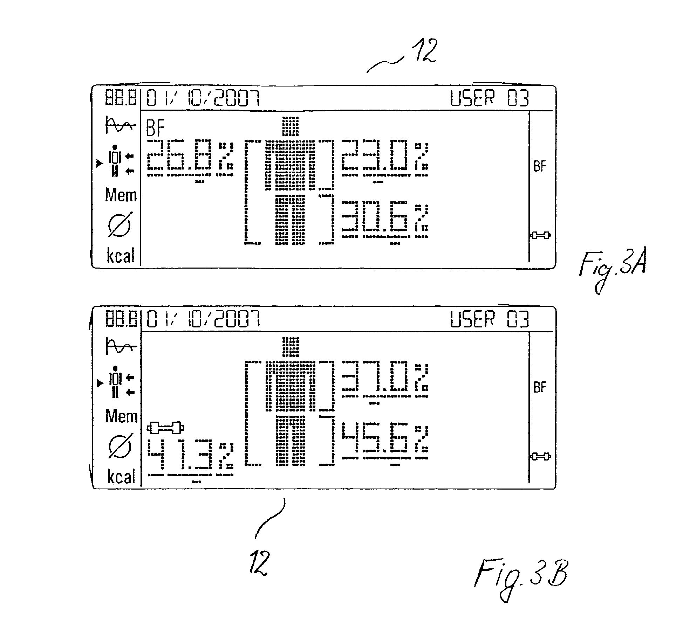

Wie die Fig. 3A und 3B zeigen, können die betreffenden Messwerte mit geeigneten Zusatzinformationen mittels der Anzeigeeinrichtung mit der Anzeigeeinheit 12 angezeigt werden, wobei die Anzeige in Fig. 3A die Körperfettanteile und in Fig. 3B Muskelmassewerte darstellt.

Zum Ermitteln des Gesamtkörperfettwertes kann in der Auswerteeinrichtung 30 z. B. in einfacher Weise eine Mittelwertbildung zwischen den beiden Teil-Körperfettwerten aus der Messung mit den Handelektroden 11, 11' und den Fußelektroden 21, 21' erfolgen, wobei auch eine gewichtete Mittelwertbildung vorgenommen werden kann.

Eine synchrone oder asynchrone Signaleinleitung über die Elektroden in den Körper kann beispielsweise unter Zeitsteuerung regelmäßig in festen oder aber in unregelmäßigen oder zufälligen Zeitabständen erfolgen, wobei als Referenz Nulldurchgänge, Scheitelwerte oder eine geeignete andere Stelle der eingespeisten Wechselspannung bzw. Signalspannung zugrunde gelegt werden kann.

Für die drahtlose Datenübertragung von der Fußmesseinheit 20 bzw. der Handmesseinheit 10 zu der Auswerteeinrichtung 30 können synchrone und/oder asynchrone Steuersignale verwendet werden, die separat und/oder moduliert auf Basis von Amplitude, Frequenz oder in Kombination abgegeben werden.

Anstelle einer Messung bei zwei oder mehr unterschiedlichen festen Frequenzen der über die Handelektroden 11, 11' bzw. Fußelektroden 21, 21' eingespeisten Messspannung kann alternativ die Messung auch kontinuierlich über ein Frequenzspektrum vorgenommen werden.

Mittels der Speichervorrichtung der Messvorrichtung kann nicht nur eine Berechnung der zeitlich versetzt eingehenden Messsignale der Handmesseinheit 10 und der

Fußmesseinheit 20 vorgenommen werden, sondern die Speichervorrichtung, insbesondere bei Ausbildung als permanente Speichervorrichtung, insbesondere bei Einbau in der Handmesseinheit 10, kann auch dazu genutzt werden, die Daten an anderer Stelle entfernt von der Messplattform der Waage auszulesen bzw. anzuzeigen, beispielsweise auch während eines Arztbesuches zur Kontrolle von Patientendaten.

Dabei können auch über bestimmte Zeiträume wiederholt erfasste Messwerte gesammelt und bewertet werden. Dabei ist es auch von Vorteil, beispielsweise zwischen der Handmesseinheit 10 und einem externen Gerät eine Datenübertragung über z. B. standardisierte Schnittstellen zu ermöglichen, wozu die Handmesseinheit 10 bei einer Ausführungsvariante ebenfalls entsprechend ausgebildet ist.

Eine weitere vorteilhafte Ausgestaltung der Messvorrichtung, insbesondere der Auswerteeinrichtung, besteht darin, dass diese mit einer Uhrenfunktion, einer Signalisierungsfunktion, beispielsweise zum Durchführen regelmäßiger Messungen, sowie auch mit einer Fehlerüberwachungsfunktion und/oder Diagnosefunktion für die Geräteüberwachung und Sicherstellung fehlerfreier Messwerte und für Wartungszwecke ausgestattet ist.

Eine weitere vorteilhafte Ausgestaltung besteht darin, dass die Messvorrichtung mit einer automatischen Abschalt-/Anschaltvorrichtung versehen ist, um einen unnötigen Stromverbrauch zu unterbinden. Dabei kann auch eine Stromsparschaltung vorgesehen sein, um eine Strom sparende Betriebsbereitschaft aufrecht zu erhalten.

Die kabellose Datenübertragungsverbindung zwischen Fußmesseinheit 20 und Handmesseinheit 10 ergibt ein kabelloses Mobilteil in Form der Handmesseinheit 10, die eine ungehinderte Mobilität ermöglicht. Zudem sind keine hinderlichen Kabelverbindungen vorhanden, die insbesondere bei in der Bewegung eingeschränkten Menschen Stolperfallen bilden können und zudem auch ungünstig bei der Aufbewahrung der Personenwaage sind. Auch ein Betrieb über eine Akkuversorgung oder eine andere netzunabhängige Stromversorgung ist möglich und bietet ein einfaches Aufladen mit elektrischer Energie.

## Patentansprüche

1. Personenwaage mit einer Messvorrichtung für einen Körperfettanteil, die eine Impedanzmesseinrichtung aufweist, wobei der Körper mit einem Messstrom, insbesondere einem einen Wechselanteil enthaltenden Strom beaufschlagt wird und eine Fußmesseinheit (20) **dadurch** gebildet ist, dass auf einer Messplattform mindestens zwei Fußelektroden (21, 21') zum Einspeisen des Stroms in den Körper angeordnet sind,
**dadurch gekennzeichnet,**
**dass** die Messvorrichtung des Weiteren eine Handmesseinheit (10) mit mindestens zwei Handelektroden (11, 11') aufweist, wobei eine Auswerteeinrichtung (30) zur Verarbeitung der von den Fußelektroden (21, 21') und von den Handelektroden (11, 11') erhaltenen Signale ausgebildet ist und die Signale von den Fußelektroden (21, 21') und/oder den Handelektroden (11, 11') drahtlos an die Auswerteeinrichtung (30) übertragen werden.

2. Personenwaage nach Anspruch 1,
**dadurch gekennzeichnet,**
**dass** zur Messung ein Wechselstrom mit mindestens zwei Frequenzen zwischen 1 kHz und 1000 kHz, insbesondere zwischen 3 kHz und 70 kHz verwendet wird.

3. Personenwaage nach Anspruch 1 oder 2,
**dadurch gekennzeichnet,**
**dass** sie auch für die Messung(en) der Knochenmasse, der Muskelmasse und/oder des Wasseranteils ausgebildet ist.

4. Personenwaage nach einem der vorhergehenden Ansprüche,
**dadurch gekennzeichnet,**
**dass** die Impedanzmesseinrichtung in Mehrleiter-, insbesondere Vierleitertechnik ausgebildet ist.

5. Personenwaage nach einem der vorhergehenden Ansprüche,
**dadurch gekennzeichnet,**
**dass** die Messungen mit den Handelektroden (11, 11') und mit den Fußelektroden (21, 21') zueinander zeitversetzt erfolgen.

6. Personenwaage nach einem der vorhergehenden Ansprüche,
**dadurch gekennzeichnet,**
**dass** die drahtlose Verbindung als Funkverbindung, Ultraschallverbindung, Infrarotverbindung oder mit Transpondertechnik oder als Kombination aus mindestens zweien dieser Verbindungen ausgebildet ist.

7. Personenwaage nach einem der vorhergehenden Ansprüche,
**dadurch gekennzeichnet,**
**dass** die Messvorrichtung zur Messung von Teil-Körperfettwerten einerseits mit der Fußmesseinheit (20) und andererseits mit der Handmesseinheit (10) ausgebildet ist.

8. Personenwaage nach Anspruch 7,
**dadurch gekennzeichnet,**
**dass** mit der Auswerteeinrichtung (30) aus den Teil-Körperfettwerten ein Gesamt-Körperfettwert berechenbar ist, und
**dass** der Gesamt-Körperfettwert und die Teil-Körperfettwerte mittels einer Anzeigevorrichtung anzeigbar sind.

9. Personenwaage nach einem der vorhergehenden Ansprüche,
**dadurch gekennzeichnet,**
**dass** die Auswerteeinrichtung (30) eine Auswerteeinheit aufweist, die zum Ermitteln und Bewerten eines ohmschen Widerstandsanteils und eines Blindwiderstandsanteils bei mindestens zwei verschiedenen Frequenzen sowohl bei der Messung mit der Handmesseinheit (10) als auch mit der Fußmesseinheit (20) ausgebildet ist.

10. Personenwaage nach Anspruch 9,
**dadurch gekennzeichnet,**
**dass** mittels der Auswerteeinheit die Teil-Körperfettwerte und/oder der Gesamtkörperfettwert in Abhängigkeit der ohmschen Anteile und der Blindwiderstandsanteile ermittelt werden.

11. Personenwaage nach einem der vorhergehenden Ansprüche,
**dadurch gekennzeichnet,**
**dass** eine Anzeigeeinheit der Anzeigevorrichtung in der Handmesseinheit angeordnet ist.
